Europäisches Patentamt

European Patent Office

Office européen des brevets

(11) Publication number: **0 530 522 A2**

## EUROPEAN PATENT APPLICATION

(21) Application number: **92113339.3**

(22) Date of filing: **05.08.92**

(51) Int. Cl.5: **C12P 13/02**, C12N 1/20,
//(C12N1/20,C12R1:22,1:01)

(30) Priority: **05.08.91 JP 195496/91**
**05.08.91 JP 195497/91**
**05.08.91 JP 195498/91**
**12.09.91 JP 233244/91**
**14.10.91 JP 264980/91**
**14.10.91 JP 264981/91**
**17.12.91 JP 333533/91**
**17.12.91 JP 333534/91**
**28.02.92 JP 43457/92**
**28.02.92 JP 43458/92**

(43) Date of publication of application:
**10.03.93 Bulletin 93/10**

(84) Designated Contracting States:
**BE DE FR GB IT NL**

(71) Applicant: **MITSUBISHI KASEI CORPORATION**
**5-2, Marunouchi 2-chome Chiyoda-ku**
**Tokyo 100(JP)**

(72) Inventor: **Morimoto, Yuuki**
**2-11-1, Naruse**
**Machida-shi, Tokyo-to(JP)**
Inventor: **Miyasaka, Kiyoyuki**
**19-404, 1-13-6, Ogawa**
**Machida-shi, Tokyo-to(JP)**
Inventor: **Sashida, Reiko**
**4-44-7, Higashiyurigaoka, Asao-ku**
**Kawasaki-shi, Kanagawa-ken(JP)**
Inventor: **Teranishi, Yutaka**
**1-14-2, Takane**
**Sagamihara-shi, Kanagawa-ken(JP)**
Inventor: **Ochiai, Masanori**
**2-15-15, Katsuradai, Midori-ku**
**Yokohama-shi, Kanagawa-ken(JP)**
Inventor: **Yamada, Kazunori**
**28-6, Umegaoka, Midori-ku**
**Yokohama-shi, Kanagawa-ken(JP)**
Inventor: **Mori, Akiko**
**2-26-9, Katsuradai, Midori-ku**
**Yokohama-shi, Kanagawa-ken(JP)**

(74) Representative: **Hansen, Bernd, Dr.**
**Dipl.-Chem. et al**
**Hoffmann, Eitle & Partner Patent- und**
**Rechtsanwälte Arabellastrasse 4 Postfach**
**81 04 20**
**W-8000 München 81 (DE)**

(54) **Process for preparing amides.**

(57) A method for preparing amides from nitriles, which method utilizes a microorganism belonging to a genus of Klebsiella, Aeromonas, Citrobacter, Agrobacterium, Rhizobium, Xanthobacter, Erwinia, Enterobacter or Streptomyces and having an ability of converting nitriles to amides, is provided. The present method enables to obtain highly purified amides which are industrially useful.

EP 0 530 522 A2

Field of the Invention

The present invention relates to a method for converting nitriles to corresponding amides by hydration utilizing an action of a microorganism. More particularly, the present invention relates to a method for biologically preparing amides characterized in that a particular microorganism is used.

Prior Art

Heretofore, a chemical process such as sulfuric acid process or copper-catalyzed process has been industrially used for production of amides starting from corresponding nitriles. A process utilizing an enzyme derived from a microorganism has been also reported recently. Advantages of the process utilizing a microorganism are that i) polymerization of starting materials and final products hardly occurs; ii) side products are relatively small; iii) reaction apparatus may be relatively small, or the like, which are attributed to use of relatively mild reaction conditions. Microorganisms having an enzyme activity which allows to produce amides from nitriles in the presence of water include Gram-positive bacteria [Japanese Patent Publication No. 21,519/1987 (Kokoku)], particularly coryneform bacteria [for example, Arthrobacter, Rhodococcus and Corynebacterium; Japanese Patent Publication Nos. 17,918/1981 (Kokoku), 2,693/1984 (Kokai), 162,193/1986 (Kokai) and 91,189/1987(Kokai)], and fungi [Fusarium; Japanese Patent Publication No. 86,889/1989 (Kokoku)]. Among Gram-negative bacteria, only Pseudomonas was reported as a microorganism having the enzyme activity [Japanese Patent Publication No. 37,951/1984 (Kokoku)].

Microorganisms are roughly classified into eucaryote (Basidiomycetes, filamentous fungi, yeasts, etc.) and prokaryote (bacteria, Cyanophyceae, Actinomycetes, etc.). Bacteria are further classified into Gram-positive organism (coryneform bacteria, Bacillus, Staphylococcus, lactic acid bacteria, acetic acid bacteria, Actinomycetes, etc.) and Gram-negative organism (Enterobacter, Pseudomonas, Vibrio, etc.) depending on their cell wall constructions, and it is recognized that they are largely different from each other taxonomically.

Furthermore, among the prokaryotic microorganisms, Actinomycetes bacteria are recognized that they form a unique group different from other bacteria because i) they have morphologically developed aerial mycelia and basal mycelia, and differentiation of tissue such as morphological differentiation of sporulation cells is also observed, although it is immature; ii) as a chemical and taxonomic characteristic, most of Actinomycetes bacteria show 62-79% of GC content in DNA and this value is higher than that of other bacteria; and iii) their phylogenetic positions based on 5S rRNA base sequence lie in an intermediate position between Gram-negative and Gram-positive bacteria [Zukai Biseibutu Hand Book, Maruzen (1990); and Japan Actinomycetes Society Bulletin (1985)]. According to Bergey's Manual of Systematic Bacteriology, Vol.4 (1984), there exist more than fifty of genera in Actinomycetes and these are classified by characteristics of aerial mycelia and basal mycelia and mode of sporulation.

Brief Description of the Invention

The present inventors have found as a result of extensive screening of microorganisms from soil that newly discovered microorganisms belonging to a genus of Klebsiella, Aeromonas, Citrobacter, Agrobacterium, Rhizobium, Xanthobacter, Erwinia, Enterobacter or Streptomyces have an ability of converting nitriles to amides, and they have established a novel method for preparing amides using said microorganisms.

Thus, the present invention provides a method for preparing amides from nitriles by an action of a microorganism, characterized in that said microorganism is a microorganism belonging to a genus of Klebsiella, Aeromonas, Citrobacter, Agrobacterium, Rhizobium, Xanthobacter, Erwinia, Enterobacter or Streptomyces and having an ability of converting nitriles to amides.

As described below, the present method enables to obtain industrially useful and highly purified amides from nitriles by utilizing a microorganism belonging to a genus of Klebsiella, Aeromonas, Citrobacter, Agrobacterium, Rhizobium, Xanthobacter, Erwinia, Enterobacter or Streptomyces.

Detailed Description of the Invention

Nitriles used as a starting material in the present invention include:
- simple nitriles such as acetonitrile, propionitrile, n-butyronitrile, and isobutyronitrile;
- α-aminonitriles such as α-aminopropionitrile, α-aminomethylthionitriles, α-aminobutyronitrile, and aminoacetonitrile;

- $\alpha$-hydroxynitriles such as lactonitrile, hydroxyacetonitrile, and $\alpha$-hydroxy-$\gamma$-methylthiobutyronitrile;
- $\beta$-aminonitriles such as amino-3-propionitrile;
- dinitriles such as malononitrile, succinonitrile, and adiponitrile;
- $\alpha$-unsaturated nitriles such as acrylonitrile, and methacrylonitrile;
- $\alpha$-benzene nitriles such as homoveratronitrile, and benzonitrile; and
- heterocyclic nitriles such as nicotinonitrile, and isonicotinonitrile.

Of these nitriles, preferred are nitriles having a carbon atom number of 2 to 4 such as acetonitrile, propionitrile, acrylonitrile, methacrylonitrile, n-butyronitrile, and isobutyronitrile. Particularly preferred is acrylonitrile.

Amides formed from the above nitriles are those corresponding to the nitrile. For example, acetamide is formed from acetonitrile, propionamide from propionitrile, and acrylamide from acrylonitrile.

Next, microorganisms used in the present invention are described below.

The microorganisms used in the present invention are not limited to particular ones, if they belong to a genus selected from the group consisting of Klebsiella, Aeromonas, Citrobacter, Agrobacterium, Rhizobium, Xanthobacter, Erwinia, Enterobacter and Streptomyces, and if they have an ability to hydrate nitriles and convert them into amides. Examples of such microorganisms are Klebsiella sp. MCI 2609, which may be hereafter abbreviated to "MCI 2609 strain"; Aeromonas sp. MCI 2614, which may be hereafter abbreviated to "MCI 2614 strain"; Citrobacter freundii MCI 2615, which may be hereafter abbreviated to "MCI 2615 strain"; Rhizobium sp. MCI 2610, which may be hereafter abbreviated to "MCI 2610 strain"; Rhizobium sp. MCI 2643, which may be hereafter abbreviated to "MCI 2643 strain"; Agrobacterium rhizogenes IAM 13570, which may be hereafter abbreviated to "IAM 13570 strain"; Agrobacterium tumefaciens IAM 13129, which may be hereafter abbreviated to "IAM 13129 strain"; Rhizobium loti IAM 13588, which may be hereafter abbreviated to "IAM 13588 strain"; Rhizobium legminosarum IAM 12609, which may be hereafter abbreviated to "IAM 12609 strain"; Rhizobium melioti IAM 12611, which may be hereafter abbreviated to "IAM 12611 strain"; Xanthobacter flavus JCM 1204, which may be hereafter abbreviated to "JCM 1204 strain"; Erwinia nigrifluens MAFF 03-01435, which may be hereafter abbreviated to "MAFF 03-01435 strain"; Enterobacter sp. MCI 2707, which may be hereafter abbreviated to "MCI 2707 strain"; and Streptomyces sp. MCI 2691, which may be hereafter abbreviated to "MCI 2691 strain". These strains have been deposited at the Fermentation Research Institute, Agency of Industrial Science and Technology, 1-3, Higashi 1 chome, Tsukuba-shi, Ibaraki-ken, 305, Japan. Among them, MCI 2609, MCI 2614, MCI 2615, MCI 2610, MCI 2643, MCI 2707 and MCI 2691 strains have been deposited under Budapest Treaty as follows.

| Strain | Accession Number | Deposition Date |
|---|---|---|
| Klebsiella sp. MCI 2609 | FERM BP-3949 | July 29, 1991 |
| Aeromonas sp. MCI 2614 | FERM BP-3950 | July 29, 1991 |
| Citrobacter freundii MCI 2615 | FERM BP-3951 | July 29, 1991 |
| Rhizobium sp. MCI 2610 | FERM BP-3952 | August 29, 1991 |
| Rhizobium sp. MCI 2643 | FERM BP-3953 | August 29, 1991 |
| Enterobacter sp. MCI 2707 | FERM BP-3955 | February 25, 1992 |
| Streptomyces sp. MCI 2691 | FERM BP-3954 | February 25, 1992 |

Among the above strains, MCI 2609, MCI 2614, MCI 2615, MCI 2610, MCI 2643, MCI 2707, and MCI 2691 are the strains isolated from natural soil by the present inventors. Bacteriological characteristics of these strains are as follows.

3

A. Klebsiella sp. MCI 2609

a) Morphological characteristics

Cultivation was carried out on Heart-Infusion agar medium at 30°C for 3 days.

| | |
|---|---|
| 1) Shape and size of cells | rod |
| 2) Pleomorphism of cells | none |
| 3) Motility | none |
| 4) Sporulation | none |
| 5) Gram stain | negative |
| 6) Acid-fast stain | negative |

b) Cultural characteristics

Characteristics of colonies after cultivation on Heart-Infusion agar medium at 30°C for 3 days are listed below.

| | |
|---|---|
| 1) Shape | circular |
| 2) Elevation | convex |
| 3) Surface | smooth |
| 4) Luster | lustrous |
| 5) Color | yellowish gray |
| 6) Optical property | opaque |
| 7) Margin | perfectly margined |

EP 0 530 522 A2

c) Physiological characteristics

```
        1)  Aerobic growth              :  +

        2)  Anaerobic growth         :  +

        3)  Catalase                 :  +

        4)  Oxidase                  :  -

        5)  O-F test                 :  F

        6)  Hydrolysis of gelatin    :  -

        7)  Litmus milk              :  acidic, coagulated

        8)  Reduction of nitrate     :  +

        9)  Denitrification          :  +

       10)  Methyl red test          :  -

       11)  VP test                  :  +

       12)  Formation of indole      :  -

       13)  Formation of H₂S         :  -

       14)  Hydrolysis of starch     :  -

       15)  Utilization of citrate   :  +
            (Christensen medium)

       16)  Utilization of inorganic nitrogen source :

                           NH₄     :  +
                           NO₃     :  +

       17)  Urease                   :  +

       18)  Decarboxylation
            of lysine                :  +

       19)  Decarboxylation
            of ornithine             :  -

       20)  DNase                    :  -

       21)  IPA reaction             :  -

       22)  Formation of pigment     :  -

       23)  Growth temperature range :  10-45°C

       24)  Growth pH range          :  4-10
```

5

## 25) Formation of acid and gas from single carbon source :

|  | Carbon source | Acid | Gas |
|---|---|---|---|
| 1 | L-arabinose | + | + |
| 2 | D-xylose | + | + |
| 3 | D-glucose | + | + |
| 4 | D-mannose | + | + |
| 5 | D-fructose | + | + |
| 6 | D-galactose | + | + |
| 7 | maltose | + | + |
| 8 | sucrose | + | + |
| 9 | lactose | + | + |
| 10 | trehalose | + | + |
| 11 | D-sorbitol | + | + |
| 12 | D-mannitol | + | + |
| 13 | glycerol | + | + |
| 14 | starch | + | – |

d) Chemical taxonomy

GC content in DNA : 59%

e) Taxonomic consideration

1) Identification in Family level

From the characteristics of the MCl 2609 strain that i) it is a Gram-negative rod, ii) it is facultatively anaerobic, iii) it utilizes glucose, iv) it is oxidase-negative, or the like, it was found that this strain belongs to Enterobacteriacae family in Facultatively Anaerobic Gram-negative Rods group described in Bergey's Manual of Systematic Bacteriology, Vol.1, as well as in Cowan and Steel, Manual for the Identification of Medical Bacteria, 2nd ed. (1974).

2) Identification in Genus and Species levels

Comparison of the MCl 2609 strain with other bacteria belonging to Enterobacteriacae family (Manual for the Identification of Medical Bacteria, 2nd ed.) reveals that this strain has the following characteristics: v) it has no motility; vi) it is positive in VP test; vii) it utilizes citrate and is urease-positive; viii) it does not decarbonate ornithine; ix) it does not decarbonate lysine; x) it utilizes inositol, or the like. Consequently, it was found that this strain belongs to Klebsiella genus.

6

Furthermore, GC content in DNA of the strain is 59%, and this value is coincident with that of <u>Klebsiella</u> (53-59%).

B. <u>Aeromonas sp.</u> MCI 2614

a) Morphological characteristics

Cultivation was carried out on Heart-Infusion agar medium at 30°C for 3 days.

| | |
|---|---|
| 1) Shape and size of cells | rod |
| 2) Pleomorphism of cells | none |
| 3) Motility | motile, polar flagella |
| 4) Sporulation | none |
| 5) Gram stain | negative |
| 6) Acid-fast stain | negative |

b) Cultural characteristics

Characteristics of colonies after cultivation on Heart-Infusion agar medium at 30°C for 3 days are listed below.

| | |
|---|---|
| 1) Shape | circular |
| 2) Elevation | convex |
| 3) Surface | smooth |
| 4) Luster | lustrous |
| 5) Color | yellowish gray |
| 6) Optical property | opaque |
| 7) Margin | perfectly margined |

c) Physiological characteristics

| 1) | Aerobic growth | : | + |
| 2) | Anaerobic growth | : | + |
| 3) | Catalase | : | + |
| 4) | Oxidase | : | + |
| 5) | O-F test | : | F |
| 6) | Hydrolysis of gelatin | : | + |
| 7) | Litmus milk | : | acidic, coagulated and peptonized |
| 8) | Reduction of nitrate | : | + |
| 9) | Denitrification | : | + |
| 10) | Methyl red test | : | - |
| 11) | VP test | : | + |
| 12) | Formation of indole | : | + |
| 13) | Formation of $H_2S$ | : | - |
| 14) | Hydrolysis of starch | : | + |
| 15) | Utilization of citrate | : | + |

8

(Christensen medium)

16) Utilization of inorganic nitrogen source :

$$NH_4 \quad : \quad +$$

$$NO_3 \quad : \quad \pm$$

17) Urease                    :  +

18) Decarboxylation
    of lysine                 :  -

19) Decarboxylation
    of ornithine              :  -

20) DNase                     :  +

21) IPA reaction              :  -

22) Formation of pigment  :  -

23) Growth temperature range  :  4-40°C

24) Growth pH range       :  5-10

## 25) Formation of acid and gas from single carbon source :

|  | Carbon source | Acid | Gas |
|---|---|---|---|
| 1 | L-arabinose | + | − |
| 2 | D-xylose | − | − |
| 3 | D-glucose | + | + |
| 4 | D-mannose | + | − |
| 5 | D-fructose | + | ± |
| 6 | D-galactose | + | + |
| 7 | maltose | + | − |
| 8 | sucrose | + | − |
| 9 | lactose | − | − |
| 10 | trehalose | + | − |
| 11 | D-sorbitol | ± | − |
| 12 | D-mannitol | + | − |
| 13 | glycerol | + | − |
| 14 | starch | + | − |

d) Chemical taxonomy

GC content in DNA : 62%

e) Taxonomic consideration

1) Identification in Family level

From the characteristics of the MCI 2614 strain that i) it is a gram-negative rod, ii) it is facultatively anaerobic, iii) it utilizes glucose, iv) it is oxidase-positive, v) it has polar flagella, or the like, it was found that this strain belongs to Vibrionacae family in Facultatively Anaerobic Gram-negative Rods group described in Bergey's Manual of Systematic Bacteriology, Vol.1, as well as in Cowan and Steel, Manual for the Identification of Medical Bacteria, 2nd ed. (1974).

2) Identification in Genus and Species levels

The MCI 2614 strain was compared with other bacteria belonging to Aeromonas, Plesiomonas and Vibrio genera in Vibrionacae family [T. Itoh, Medical Technology, vol.12, p.799 (1984)]. As shown in the table below, characteristics of this strain showed good coincidence with those of Aeromonas, and therefore, the strain was identified to be Aeromonas sp.

Furthermore, GC content in DNA of the strain is 62%, and this value is coincident with that of Aeromonas (58-62%).

| | MCI 2614 | Aeromonas hydrophila | Aeromonas sorbria | Plesiomonas shigelloides | Vibrio cholerae |
|---|---|---|---|---|---|
| TSI agar | | | | | |
| LIM agar | | | | | |
| slant | yellow (red) | yellow (red) | yellow (red) | red | yellow |
| stab | yellow | yellow | yellow | yellow | yellow |
| gas | + | + | + | - | - |
| hydrogen sulfide | - | - | - | - | - |
| lysine | - | - | - | + | + |
| indole | + | d | d | + | + |
| motility | + | + | + | + | + |
| oxidase | + | + | + | + | + |
| inositol | - | - | - | + | - |
| mannitol | + | + | + | - | + |
| VP test | + | + | d | - | d |
| decarboxylation of ornithine | - | - | - | d | + |
| hydrolysis of arginine | + | d | d | + | - |
| GC content in DNA | 62% | 58-62% | 58-60% | 51% | 47-49% |

d indicates that utilization ability differs from species to species.

## C. Citrobacter freundii MCI 2615

a) Morphological characteristics

Cultivation was carried out on Heart-Infusion agar medium at 30°C for 3 days.

| | |
|---|---|
| 1) Shape and size of cells | rod |
| 2) Pleomorphism of cells | none |
| 3) Motility | motile, peripheric flagella |
| 4) Sporulation | none |
| 5) Gram stain | negative |
| 6) Acid-fast stain | negative |

b) Cultural characteristics

Characteristics of colonies after cultivation on Heart-Infusion agar medium at 30°C for 3 days are listed below.

| | |
|---|---|
| 1) Shape | circular |
| 2) Elevation | convex |
| 3) Surface | smooth |
| 4) Luster | lustrous |
| 5) Color | yellowish gray |
| 6) Optical property | opaque |
| 7) Margin | undulate |

c) Physiological characteristics

| | | | |
|---|---|---|---|
| 1) | Aerobic growth | : | + |
| 2) | Anaerobic growth | : | + |
| 3) | Catalase | : | + |
| 4) | Oxidase | : | − |
| 5) | O-F test | : | F |

6) Hydrolysis of gelatin : -

7) Litmus milk : acidic

8) Reduction of nitrate : +

9) Denitrification : +

10) Methyl red test : -

11) VP test : -

12) Formation of indole : -

13) Formation of $H_2S$ : +

14) Hydrolysis of starch : -

15) Utilization of citrate : +
    (Christensen medium)

16) Utilization of inorganic nitrogen source :

$NH_4$ : +

$NO_3$ : -

17) Urease : +

18) Decarboxylation
    of lysine : -

19) Decarboxylation
    of ornithine : -

20) DNase : -

21) IPA reaction : -

22) Formation of pigment : -

23) Growth temperature range : 10-45°C

24) Growth pH range : 4-10

## 25) Formation of acid and gas from single carbon source :

| | Carbon source | Acid | Gas |
|---|---|---|---|
| 1 | L-arabinose | + | + |
| 2 | D-xylose | + | + |
| 3 | D-glucose | + | + |
| 4 | D-mannose | + | + |
| 5 | D-fructose | + | + |
| 6 | D-galactose | + | - |
| 7 | maltose | + | + |
| 8 | sucrose | + | + |
| 9 | lactose | + | + |
| 10 | trehalose | + | + |
| 11 | D-sorbitol | + | + |
| 12 | D-mannitol | + | + |
| 13 | glycerol | + | + |
| 14 | starch | ± | - |

d) Chemical taxonomy

GC content in DNA : 52%

e) Taxonomic consideration

1) Identification in Family level

From the characteristics of the MCI 2615 strain that i) it is a gram-negative rod, ii) it is facultatively anaerobic, iii) it utilizes glucose, iv) it is oxidase-negative, or the like, it was found that this strain belongs to Enterobacteriacae family in Facultatively Anaerobic Gram-negative Rods group described in Bergey's Manual of Systematic Bacteriology, Vol.1, as well as in Cowan and Steel, Manual for the Identification of Medical Bacteria, 2nd ed. (1974).

2) Identification in Genus and Species levels

The MCI 2615 strain was compared with other bacteria belonging to Enterobacteriacae family and forming hydrogen sulfide [Cowan and Steel, Manual for the Identification of Medical Bacteria, 2nd ed. (1974), and The Prokaryotes, Vol.2]. As shown in the table below, characteristics of this strain showed good coincidence with those of Citrobacter freundii with the exception of utilization of inositol. The difference in the utilization of inositol was considered due to an intra-species variation, and therefore, the strain was identified to be Citrobacter freundii.

Furthermore, GC content in DNA of the strain is 52%, and this value is coincident with that of Citrobacter freundii (50-53%).

|  | MCI 2615 | Citrobacter freundii | Salmonella genus | Proteus genus |
|---|---|---|---|---|
| hydrogen sulfide | + | + | + | + |
| indole | - | - | - | d |
| decarboxylation of ornithine | - | - | + | d |
| decarboxylation of lysine | - | - | + | - |
| urease | + | d | - | + |
| liquefying of gelatin | - | - | - | + |
| utilization of citrate | + | + | d | d |
| lactose | + | + | - | - |
| D-mannitol | + | + | + | - |
| D-mannose | + | + | + | - |
| L-rhamnose | + | + | + | - |
| inositol | + | - | - | - |
| GC content in DNA | 52% | 50-53% | 50-53% | 38-41% |

d indicates that utilization ability differs from species to species.

D. Rhizobium sps. MCI 2610 and MCI 2643

a) Morphological characteristics

Cultivation was carried out on a conventional agar medium at 30°C for 3 days.

|  | MCI 2610 | MCI 2643 |
|---|---|---|
| 1) Shape and size of cells | rod | rod |
| 2) Pleomorphism of cells | none | none |
| 3) Motility | motile, peripheric flagella | motile, peripheric flagella |
| 4) Sporulation | none | none |
| 5) Gram stain | negative | negative |
| 6) Acid-fast stain | negative | negative |

b) Cultural characteristics

Characteristics of colonies after cultivation on a conventional agar medium at 30°C for 3 days are listed below.

|  | MCI 2610 | MCI 2643 |
|---|---|---|
| 1) Shape | circular | circular |
| 2) Elevation | convex | convex |
| 3) Surface | smooth | smooth |
| 4) Luster | lustrous | lustrous |
| 5) Color | yellowish white | yellowish white |
| 6) Optical property | opaque | opaque |
| 7) Margin | perfectly margined | perfectly margined |
| 8) Viscosity | slightly viscous | viscous |

c) Physiological characteristics

|  |  |  | MCI 2610 | MCI 2643 |
|---|---|---|---|---|
| 1) | Aerobic growth | : | + | + |
| 2) | Anaerobic growth | : | − | − |
| 3) | Catalase | : | + | + |
| 4) | Oxidase | : | + | + |
| 5) | O-F test | : | O | O |
| 6) | Hydrolysis of gelatin | : | − | − |
| 7) | Litmus milk | : | not changed | not changed |
| 8) | Reduction of nitrate | : | + | + |
| 9) | Denitrification | : | ± | + |
| 10) | Methyl red test | : | − | + |
| 11) | VP test | : | − | − |
| 12) | Formation of indole | : | − | − |
| 13) | Formation of $H_2S$ | : | − | − |
| 14) | Hydrolysis of starch | : | − | − |
| 15) | Utilization of citrate (Christensen medium) | : | − | − |
| 16) | Utilization of inorganic nitrogen source : | | | |
| | $NH_4$ | : | + | + |
| | $NO_3$ | : | − | + |
| 17) | Urease | : | + | + |
| 18) | Decarboxylation of lysine | : | ± | ± |
| 19) | Decarboxylation of ornithine | : | + | + |
| 20) | Hydrolysis of arginine | : | − | − |
| 21) | DNase | : | − | − |

```
22) Phosphatase                 :        +           +

23) Decomposition
      of tyrosine               :        +           +

24) Decomposition
      of chitin                 :        -           -

25) Decomposition
      of Tween 80               :        -           -

26) Formation of
      3-ketolactose            :        -           -

27) Formation of pigment   :   brown pigment    -

28) Growth temperature range :   10-40°C      10-40°C

29) Growth pH range            :        5-10         5-10

30) Resistance to NaCl         :     growth       growth
                                     at 2%        at 2%
```

31) Formation of acid from single carbon source (cultivated for two weeks):

| | Carbon source | MCI 2610 | MCI 2643 |
|---|---|---|---|
| 1 | L-arabinose | + | + |
| 2 | D-xylose | + | + |
| 3 | D-glucose | + | + |
| 4 | D-mannose | + | + |
| 5 | D-fructose | + | + |
| 6 | D-galactose | + | + |
| 7 | maltose | + | + |
| 8 | sucrose | + | + |
| 9 | lactose | − | + |
| 10 | trehalose | + | + |
| 11 | D-sorbitol | + | + |
| 12 | D-mannitol | + | + |
| 13 | glycerol | + | + |
| 14 | starch | − | − |
| 15 | cellobiose | + | ± |
| 16 | L-rhamnose | + | + |
| 17 | raffinose | − | − |
| 18 | melezitose | − | − |
| 19 | adonitol | − | ± |
| 20 | dulcitol | − | − |
| 21 | erythritol | + | + |
| 22 | salicin | + | − |
| 23 | ethanol | − | + |

## 32) Utilization of organic acid (cultivated for two weeks):

| | Organic acid | MCI 2610 | MCI 2643 |
|---|---|---|---|
| 1 | acetic acid | + | - |
| 2 | benzoic acid | - | - |
| 3 | citric acid | + late | + |
| 4 | malonic acid | - | + |
| 5 | propionic acid | - | - |
| 6 | pyruvic acid | + | + |
| 7 | L-succinic acid | - | + |
| 8 | malic acid | - | + |
| 9 | gluconic acid | - | - |
| 10 | adipic acid | - | - |

d) Chemical taxonomy

| | MCI 2610 | MCI 2643 |
|---|---|---|
| 1) GC content in DNA<br>2) Ubiquinone | 66%<br>Q-10 | 63%<br>Q-10 |
| 3) Fatty acid in cell body | $C_{18:1}$<br>$3OH\text{-}C_{14:0}$<br>$3OH\text{-}C_{16:0}$<br>$3OH\text{-}C_{18:0}$ | $C_{18:1}$<br>$3OH\text{-}C_{14:0}$<br>$3OH\text{-}C_{16:0}$<br>$3OH\text{-}C_{18:0}$ |

e) Taxonomic consideration of MCI 2610 strain

1) Identification in Family level

The MCI 2610 strain is an aerobic and gram-negative rod lacking endospore in the cell and having a small number of peripheric flagella, can utilize a number of sugars, and produces extracellular polysaccharides.

The results of the chemical taxonomy revealed that this strain had high GC content of 66% and quinone system of ubiquinone Q-10. Furthermore, the strain has a fatty acid composition rich in straight chain and C18:1 of fatty acids, contains mainly 30H-C14:00 as a hydroxy fatty acid, and does not contain 2-hydroxy fatty acid. From these bacteriological characteristics of the MCI 2610 strain, it was found that this strain belongs to Rhizobiaceae family described in Bergey's Manual of Systematic Bacteriology, 1st ed., p.234 (1984).

2) Identification in Genus level

According to Bergey's Manual of Systematic Bacteriology, 1st ed., p.234-256 (1984), Rhizobiaceae family contains four genera, Rhizobium, Bradyrhizobium, Agrobacterium, and Phyllobacterium, and these genera are identified by the following characteristics:

|  | Rhizobium | Bradyrhizobium | Agrobacterium | Phyllobacterium | MCI 2610 |
|---|---|---|---|---|---|
| Position of flagella* | pol or per | pol | per | pol | per |
| Formation of root nodules | + | + | - | - | / |
| Formation of gall on leaf | - | - | - | + | / |
| Nitrogenase | + | + | - | - | / |
| Hypertrophy of host | - | - | + | - | / |
| 3-Ketolactose | - | - | ± | | - |
| Fast growth on YMA | + | - | + | + | + |
| Alkalinization of sugars | - | + | - | - | - |
| Formation of $H_2S$ | ± | - | - | / | - |
| Requirement of biotin | + | + | - | / | - |
| Extracellular polysaccharide $\beta$-2-bound glucan | + | + | + | / | / |
| GC content (mol %) | 59-64 | 61-65 | 57-63 | 60-61 | 66 |

* pol = polar and per = peripheric

Comparison of the characteristics of the MCI 2610 strain with those of four genera suggests that this strain is close to Rhizobium, Agrobacterium or Bradyrhizobium. However, a genus which has characteristics perfectly coincident with those of the MCI 2610 strain is not found in the genera in Rhizobiaceae family as described below. The MCI 2610 strain has high GC content of 66%, and this value is different from those of Rhizobium (59-64%) and Agrobacterium (57-63%) and rather close to that of Bradyrhizobium (61-65%). Since, however, the present strain has peripheric flagella and is a fast-growth bacterium on the below-described YMA medium, this strain differs from bacteria belonging to Bradyrhizobium genus. The bacteria of Bradyrhizobium genus have polar flagella and late-growth characteristics on the YMA medium.

Next, comparison of hydroxy fatty acids, which are a group of fatty acids in cell body, was performed. The hydroxy fatty acids are considered to be a particularly effective indicator for classification and identification of Gram-negative bacteria. According to Institute for fermentation, Osaka Research Communication, Vol. 15, pp. 57-75 (1991), bacteria belonging to Rhizobiaceae show a pattern of hydroxy fatty acids characteristic of species or subspecies, and they are divided into nine groups based on the composition of hydroxy fatty acids other than commonly existing $3OH-C_{14:0}$ fatty acid. Accordingly, fatty acid pattern of the present strain was compared with those of the nine groups as shown in the table below. From the comparison, it was found that the present strain has the following characteristics: i) it does not contain 2-hydroxy fatty acid; and ii) it contains a small amount of $3OH-C_{18:0}$ and $3OH-C_{16:0}$. Thus, the pattern of the present strain is similar to those of R.meliloti, R.fredii and R.galegae, and different from those of Agrobacterium bacteria.

From the above results, it appears reasonable to consider that the present MCI 2610 strain belongs to Rhizobium genus. Accordingly, the present strain was identified to be Rhizobium sp.

Differential characteristics of the species in Rhizobiaceae

| Group | Genus or Species | 3-Ketolactose formation | 2-OH fatty acids | 3-OH 12:0 | 3-OH i-13:0 | 3-OH i-15:0 | 3-OH ai-15:0 | 3-OH 16:0 | 3-OH 18:0 |
|---|---|---|---|---|---|---|---|---|---|
| 1 | Bradyrhizobium | - | - | + | - | - | - | - | - |
| 2 | R.leguminosarum | - | - | - | - | - | + | + | + |
| 3 | R.meliloti and R.fredii | - | - | - | - | - | - | ± | + |
| 4 | R.galegae | - | - | - | - | - | - | + | + |
| 5 | R.loti | - | - | + | + | - | - | + | + |
| 6 | Agrobacterium biovar 1 | + | - | - | - | - | - | + | - |
| 7 | Agrobacterium biovar 2 | - | + | - | - | + | - | + | + |
| 8 | Agrobacterium biovar 3 | - | + | - | - | - | - | + | + |
| 9 | A.rubi | - | - | - | - | - | - | + | - |
| | MCI 2610 | - | - | - | - | - | - | + | + |

f) Taxonomic consideration of MCI 2643 strain

1) Identification in Family level

The MCI 2643 strain is an aerobic and gram-negative rod lacking endospore in the cell and having a small number of peripheric flagella, and can utilize a number of sugars, and produces extracellular polysaccharides.

The results of the chemical taxonomy revealed that this strain had high GC content of 63% and quinone system of ubiquinone Q-10. Furthermore, the strain has a fatty acid composition rich in straight chain and C18:1 of fatty acids, contains mainly 30H-C14:00 as a hydroxy fatty acid, and does not contain 2-hydroxy fatty acid. From these bacteriological characteristics of the MCI 2643 strain, it was found that this strain belongs to Rhizobiaceae family as the MCI 2610 strain does.

2) Identification in Genus level

Next, comparison of hydroxy fatty acids, which are a group of fatty acids in cell body, was performed. The hydroxy fatty acids are considered to be a particularly effective indicator for classification and identification of Gram-negative bacteria. According to Institute for fermentation, Osaka Research Communication, Vol. 15, pp. 57-75 (1991), bacteria belonging to Rhizobiaceae show a pattern of hydroxy fatty acids characteristic of species or subspecies, and they are divided into nine groups based on the composition of hydroxy fatty acids other than commonly existing 30H-$C_{14:0}$ fatty acid. Accordingly, fatty acid pattern of the present strain was compared with those of the nine groups as shown in the table below. From the comparison, it was found that the present strain has the following characteristics: i) it does not contain 2-hydroxy fatty acid; and ii) it contains a small amount of 30H-$C_{18:0}$ and 30H-$C_{16:0}$. Thus, the pattern of the present strain is similar to those of R.meliloti, R.fredii and R.galegae, and different from those of Agrobacterium bacteria.

From the above results, it appears reasonable to consider that the present MCI 2643 strain belongs to Rhizobium genus. Accordingly, the present strain was identified to be Rhizobium sp.

Differential characteristics of the species in Rhizobiaceae

| Group | Genus or Species | 3-Ketolactose formation | 2-OH fatty acids | 3-OH 12:0 | 3-OH i-13:0 | 3-OH i-15:0 | 3-OH ai-15:0 | 3-OH 16:0 | 3-OH 18:0 |
|---|---|---|---|---|---|---|---|---|---|
| 1 | Bradyrhizobium | - | - | + | - | - | - | - | - |
| 2 | R.leguminosarum | - | - | - | - | - | + | + | + |
| 3 | R.meliloti and R.fredii | - | - | - | - | - | - | ± | + |
| 4 | R.galegae | - | - | - | - | - | - | + | + |
| 5 | R.loti | - | - | + | + | - | - | + | + |
| 6 | Agrobacterium biovar 1 | + | - | - | - | - | - | + | - |
| 7 | Agrobacterium biovar 2 | - | + | - | - | + | - | + | + |
| 8 | Agrobacterium biovar 3 | - | + | - | - | - | - | + | + |
| 9 | A.rubi | - | - | - | - | - | - | + | - |
| | MCI 2643 | - | - | - | - | - | - | + | + |

E. Enterobacter sp. MCI 2707

a) Morphological characteristics

Cultivation was carried out on a conventional agar medium at 30°C for 24 hours.

| | |
|---|---|
| 1) Shape and size of cells | short rod |
| 2) Pleomorphism of cells | none |
| 3) Motility | motile, peripheric flagella |
| 4) Sporulation | none |
| 5) Gram stain | negative |
| 6) Acid-fast stain | negative |

b) Cultural characteristics

Characteristics of colonies after cultivation on a conventional agar medium at 30°C for 2 days are listed below.

| | |
|---|---|
| 1) Shape | circular |
| 2) Elevation | convex |
| 3) Surface | smooth |
| 4) Luster | lustrous |
| 5) Color | yellowish gray |
| 6) Optical property | opaque |
| 7) Margin | perfectly margined |

c) Physiological characteristics

```
1)  Aerobic growth        :  +

2)  Anaerobic growth      :  +

3)  Catalase              :  +

4)  Oxidase               :  -

5)  O-F test              :  F
```

6) Hydrolysis of gelatin : -

7) Litmus milk : acidic,

        not coagulated

8) Reduction of nitrate : +

9) Denitrification : +

10) Methyl red test : -

11) VP test : +

12) Formation of indole : -

13) Formation of $H_2S$ : -

14) Hydrolysis of starch : -

15) Utilization of citrate : +
    (Christensen medium)

16) Utilization of inorganic nitrogen source :

               $NH_4$ : +

               $NO_3$ : +

17) Urease : -

18) Decarboxylation
    of lysine : +

19) Decarboxylation
    of ornithine : +

20) Hydrolysis
    of arginine : -

21) Deamination
    of tryptophan : -

22) DNase : -

23) β-Galactosidase : +

24) Decomposition
    of Tween 80 : -

25) Formation of pigment : -

26) Growth temperature range : 9-45°C

27) Growth pH range : 4-10

28) Formation of acid and gas from single carbon source :

|  | Carbon source | Acid | Gas |
|---|---|---|---|
| 1 | L-arabinose | + | + |
| 2 | D-xylose | + | + |
| 3 | D-glucose | + | + |
| 4 | D-mannose | + | + |
| 5 | D-fructose | + | + |
| 6 | D-galactose | + | + |
| 7 | maltose | + | + |
| 8 | sucrose | + | + |
| 9 | lactose | + | + |
| 10 | trehalose | + | + |
| 11 | D-sorbitol | + | + |
| 12 | D-mannitol | + | + |
| 13 | inositol | + | + |
| 14 | glycerol | + | - |
| 15 | starch | ± | - |
| 16 | rhamnose | + | + |
| 17 | raffinose | + | + |
| 18 | melibiose | + | + |

d) Chemical taxonomy

| 1) Isoprenoid quinone | Q-8, MK-8 |
|---|---|
| 2) GC content in DNA | 57 mole % |

e) Taxonomic consideration

1) Identification in Family level

From the characteristics of the MCI 2707 strain that i) it is a gram-negative rod, ii) it is facultatively anaerobic, iii) it utilizes glucose, iv) it is oxidase-negative, v) it has peripheric flagella, or the like, it was found that this strain belongs to Enterobacteriacae family in Facultatively Anaerobic Gram-negative Rods group described in Bergey's Manual of Systematic Bacteriology, Vol.1.

2) Identification in Genus level

Comparison of the characteristics of the MCI 2707 strain with those of bacteria belonging to other genera in Enterobacteriacae family (Bergey's Manual of Systematic Bacteriology, Vol.1) revealed that this strain had the following characteristics: i) it has motility; ii) it is positive in VP test; iii) it is urease-negative; iv) it decarbonates ornithine; v) it is DNase-negative; vi) it produces an acid from sorbitol, or the like. Consequently, it was found that this strain belongs to Enterobacter genus.

Furthermore, GC content in DNA of the strain is 57%, and this value is coincident with that of Enterobacter (52-60%).

3) Identification in Species level

At present, seven species are described in Enterobacter genus in Bergey's Manual of Systematic Bacteriology, Vol.1. Comparison of the MCI 2707 strain with these bacteria suggested that this strain is a close-related species to Enterobacter aerogenes as shown in the table below. However, GC content in DNA of the strain is 57%, which differs from that of E. aerogenes (53-54%) by about 3%. As shown in the table below, E. sakazakii and E. agglomerans are known to be a species having the GC content of 57%. However, the physiological characteristics of these species are clearly different from those of the present strain. Accordingly, the MCI 2707 strain was identified to be Enterobacter sp.

Comparable data of the MCI 2707 strain with various species belonging to Enterobacter genus are shown in the following table. In the table, + indicates perfect positive, (+) indicates more than 89% of positive, d indicates 10-80% of positive, and - indicates perfect negative.

26

| | E.clo acae | E.saka zakii | E.agglo merans | E.aero genes | E.gerg oviae | E.inter medium | E.amni genus | MCI 2707 |
|---|---|---|---|---|---|---|---|---|
| urease | - | - | - | - | + | - | - | - |
| liquefying of gelatin | + | (+) | (+) | d | - | - | - | - |
| decarboxylation of amino acid | | | | | | | | |
|   lysine | - | - | - | + | + | - | - | + |
|   ornithine | + | + | - | + | + | + | + | + |
|   arginine | + | + | - | - | - | - | + | - |
| production of acid | | | | | | | | |
|   sorbitol | + | - | d | + | - | + | d | + |
|   sucrose | + | + | d | + | + | d | d | + |
|   raffinose | (+) | + | d | + | + | + | + | + |
| utilization of citrate | + | + | (+) | + | + | + | + | + |
| formation of indole | - | d | d | - | - | - | - | - |
| formation of yellow pigment | - | + | d | - | - | - | - | - |
| GC content in DNA (mole %) | 52-54 | 57 | 53-58 | 53-54 | 60 | | 60 | 57 |

## F. Streptomyces sp. MCI 2691

a) Morphological characteristics

The cells were cultivated at 27°C for 14 days using starch-inorganic salts agar medium and yeast extract-malt extract agar medium as a medium for sporulation, and thereafter, development of the culture was observed. Color of colonies was white-gray, basal mycelia elongated with branching, and fragmentation was not observed. Long chain of arthrospores were formed on aerial mycelia and the majority of spore chains were linear. A minority of hooky spore chains having slight curvature at their terminals were also observed. Surface of the culture was smooth.

b) Cultural characteristics

Growth characteristics of the MCI 2691 strain after cultivation on various media defined by ISP (International Streptomyces Project) at 27°C for 14 days were shown below. In the below description, G indicates Growth, RC indicates Reverse Color, AM indicates Aerial Mycelium, SP indicates Soluble Pigment, and S indicates Sporulation.
    1) Sucrose-nitrate agar medium
        G :     poor, white-gray
        RC :    yellowish white
        AM :    poor, powdery, white
        SP :    not formed
        S :     vigorous, linear or hooky
    2) Glucose-asparagine agar medium
        G :     good, gray

RC :     yellowish white
AM :    rich, powdery, white
SP :     not formed
S :      vigorous, linear

3) Glycerol-asparagine agar medium (ISP 5)
    G :      good, yellowish white
    RC :     yellowish white - pale yellow-brown
    AM :    moderate, powdery
    SP :     not formed
    S :      not formed

4) Starch-inorganic salts agar medium (ISP 4)
    G :      good, white - light brown-gray
    RC :     pale yellow-brown
    AM :    vigorous, velvety, white
    SP :     not formed
    S :      vigorous, linear

5) Tyrosine agar medium (modification by Okanishi)
    G :      good, pale yellow-brown
    RC :     pale yellow-brown
    AM :    moderate, powdery, white
    SP :     brown
    S :      not formed

6) Conventional agar medium
    G :      good, pale yellow-brown
    RC :     pale yellow-brown
    AM :    not formed
    SP :     not formed
    S :      not formed

7) Yeast ext.-malt ext. agar medium (ISP 2)
    G :      good, gray
    RC :     pale yellow-brown
    AM :    rich, powdery, gray
    SP :     not formed
    S :      vigorous, linear or hooky

8) Oatmeal agar medium (ISP 8)
    G :      moderate, gray
    RC :     pale yellow-brown
    AM :    moderate, powdery, white
    SP :     not formed
    S :      vigorous, linear

9) Bennett agar medium
    G :      good, white-gray
    RC :     pale yellow-brown
    AM :    rich, velvety-powdery, white
    SP :     not formed
    S :      moderate, linear or hooky

10) Calcium malate agar medium
    G :               moderate, white-gray
    RC :             yellowish white
    AM :            moderate, powdery, white
    SP :             not formed
    S :              moderate, linear
    Utilization of calcium malate :    positive

c) Physiological characteristics

1) Growth temperature range                           :  9-40°C

2) Growth pH range                                    :  4-10

3) Liquefying of gelatin                              :  +

4) Hydrolysis of starch                               :  +

5) Coagulation of skim milk                           :  -

                    Peptonization                     :  +

6) Formation of melanin-like pigment
   (on peptone-yeast ext.-iron agar
   medium, tryptone-yeast ext. agar
   medium and tyrosine agar medium)                   :  +

7) Reduction of nitrate                               :  +

8) Resistance to NaCl                        :  growth at 7%


9) Utilization of carbon sources :

   (A little growth was also observed on a medium not
   supplemented with any carbon source.)

| Carbon source | Utilization |
|---|---|
| 1    D-glucose | + |
| 2    L-arabinose | + |
| 3    D-xylose | + |
| 4    D-fructose | + |
| 5    sucrose | − |
| 6    L-rhamnose | − |
| 7    raffinose | − |
| 8    inositol | ± |
| 9    D-mannitol | − |

+: utilize,  ±: doubtful in utilization,
−: not utilize

d) Component in cell body

Type of amino acid in cell wall of the MCI 2691 strain was confirmed to be cell wall type I because L,L-diaminopimeric acid was detected in hydrolysate of whole cell body. In addition, glucose and ribose were detected in the hydrolysate of whole cell body, but a characteristic pattern in the cell wall sugar composition was not observed.

e) Taxonomic consideration

From the results described above, it was found that the present MCI 2691 strain belongs to Streptomyces genus. In order to identify its species, reference to species described in ISP was performed, and it was suggested that this strain is a species similar to Streptomyces tanashiensis, S. venezuelae and S. showdoensis described in Nonomura, J. Ferment. technol., Vol.52, No.2, pp.78-92 (1974); E. B. Shirling and D. Gottlieb, Int. J. Syst. Bact., Vol.18, No.4, pp.379-380 (1968), Vol.19, No.4, pp.491-492 (1969), Vol.22, No.4, pp.352-354 and pp.374-375 (1972). However, several differences were observed in cultural and physiological characteristics as shown in the table below. Consequently, the MCI 2691 strain was identified to be Streptomyces sp.

The differences between the MCI 2691 strain and various strains are shown in the following table.

| | S.tanashiensis | S.venezuelae | S.zaomvceticus | S.showdoensis | MCI 2691 |
|---|---|---|---|---|---|
| Sporulation on various media: | | | | | |
| Glucose-asparagine agar medium | poor | poor | poor | poor | vigorous |
| Glycerol-asparagine agar medium | vigorous | vigorous | vigorous | vigorous | none |
| Starch-inorganic salts agar medium | vigorous | vigorous | none | vigorous | vigorous |
| Yeast ext.-malt ext. agar medium | vigorous | poor | vigorous | vigorous | vigorous |
| Formation of melanin -like pigment on tyrosine agar medium | − | + | + | + | + |
| Resistance to NaCl | 4% | ND | ND | 4% | 7% |
| Utilization of carbon sources: | | | | | |
| D-glucose | + | + | + | + | + |
| L-arabinose | + | + | + | ± | + |
| D-xylose | + | + | + | + | + |
| D-fructose | ± | + | − | + | + |
| sucrose | − | − | − | ± | − |
| L-rhamnose | − | + | − | − | − |
| raffinose | − | − | − | − | − |
| inositol | − | − | − | − | ± |

Microbiological hydration reaction of nitriles according to the present invention is essentially similar to that of a known method except that a particular microorganism is used. Accordingly, the term "convert nitriles to corresponding amides by hydration utilizing an action of a microorganism" herein includes an embodiment wherein the microorganism is cultivated in the presence of nitriles as well as an embodiment wherein nitriles are contacted with a cultivated medium, cell body or treated material thereof after cultivation of the microorganism. Furthermore, it also includes an embodiment wherein the cell body of the microorganism or an enzyme produced intracellularly or extracellularly by the microorganism is immobilized and used in the reaction.

Cultivation of the microorganism in the present invention may be carried out conventionally. Medium used in the present invention is a conventional one containing, at a suitable ratio, a carbon source such as glucose, glycerol, millet jelly or starch, an inorganic nitrogen source such as ammonium sulfate or ammonium nitrate, an organic nitrogen source such as soy flour, yeast extract, peptone or urea, as well as an inorganic salt such as phosphate, sodium, potassium or magnesium. Furthermore, nitriles such as acetonitrile or amides such as acrylamide for inducing a desired enzyme, and inorganic salts such as iron, zinc or cobalt ion necessary for the enzyme activity may be preferably added to the medium. Cultivation is carried out at temperature of 20-37°C for 1-5 days under the medium pH of 5-10.

Although it is possible to convert nitriles into amides by cultivating the microorganism in the presence of said nitriles which are desired to be hydrated, the following method is preferred.

Thus, the microorganism is cultivated according to the above-mentioned method, and cell bodies are collected from the cultivated medium by centrifugation. The cell bodies are suspended in a physiological saline or a buffered solution such as phosphate or tris buffer (pH 4-11), the desired nitriles such as acrylonitrile are added to the suspension, and it may be maintained under a suitable temperature condition, for example at freezing-point to 60°C. In this case, it is also possible to portionwisely add the nitriles according to the progress of the reaction.

An enzyme may be purified from cell bodies in the culture or supernatant of the culture, which is cultivated as described above, by means of destruction, ammonium sulfate precipitation, ion exchange chromatography, gel filtration chromatography, hydrophobic chromatography, and the like. Subsequently, the resultant enzyme may be used to carry out the above reaction.

Furthermore, the cell bodies or enzyme obtained in the above method may be conjugatedly immobilized in a gel such as polyacrylamide, photo cross-linking resin, agar, or carrageenan, and may be reacted with nitriles in a reactor equipped with a stirring apparatus under a suitable pH and temperature condition as described above. Alternatively, the gel may be filled in a column and reacted with nitriles by allowing a solution containing nitriles to pass through the column.

Amides obtained by the reaction can be used as an aqueous solution without any post-treatment, or as a powder by concentrating it using a method such as membrane concentration or spray-drying concentration. It is also possible to further increase their purity by a method using an activated charcoal, ion exchange resin, ion exchange membrane, and the like.

The present invention is further illustrated by the following examples, but should not be construed to be limited by them. Modified embodiments of them are included within the scope of the present invention as far as they do not depart from the spirit of the present invention. In the following examples, quantification of nitriles and amides was carried out by high performance liquid chromatography.

Example 1

Klebsiella sp. MCI 2609 strain was cultivated in an aerobic condition at 30°C for 3 days in a medium containing 0.4% glycerol, 0.2% yeast extract, 0.05% polypeptone, 0.001% $FeSO_4 \cdot 7H_2O$, 0.001% $CoCl_2 \cdot 6H_2O$, 0.2% NaCl, 0.04% $MgSO_4 \cdot 7H_2O$, 0.25% $K_2HPO_4$, and 0.025% acrylamide. After the cultivation, the cell bodies were isolated from the culture by centrifugation, washed with physiological saline, and suspended in a phosphate buffer (pH 7.0, 0.1M). An aliquot (0.8 ml) of the suspension was combined with 7% aqueous acrylonitrile solution (0.2 ml), and allowed to react at 30°C for one hour. Acrylonitrile in the reaction mixture was thoroughly converted into acrylamide, which was produced in 1.88%.

Examples 2-12

The reaction in Example 1 was repeated using a similar method except that Aeromonas sp. MCI 2614 (Example 2), Citrobacter freundii MCI 2615 (Example 3), Rhizobium sp. MCI 2610 (Example 4), Rhizobium sp. MCI 2643 (Example 5), Agrobacterium rhizogenes IAM 13570 (Example 6), Agrobacterium tumefaciens IAM 13129 (Example 7), Rhizobium loti IAM 13588 (Example 8), Rhizobium legminosarum IAM 12609 (Example 9), Rhizobium melioti IAM 12611 (Example 10), Enterobacter sp. MCI 2707 (Example 11), or Streptomyces sp. MCI 2691 (Example 12) was used instead of the MCI 2609 strain in Example 1. Production of acrylamide in the reaction mixture was confirmed in all cases.

Example 13

Xanthobacter flavus JCM 1204 strain was cultivated in an aerobic condition at 30°C for 2 days in a medium containing 0.4% glycerol, 0.2% yeast extract, 0.05% polypeptone, 0.001% $FeSO_4 \cdot 7H_2O$, 0.001% $CoCl_2 \cdot 6H_2O$, 0.2% NaCl, 0.04% $MgSO_4 \cdot 7H_2O$, 0.25% $K_2HPO_4$, and 0.1% crotonamide. The culture (2 ml) was centrifuged to isolate the cell bodies, and then they were washed with physiological saline and suspended in a phosphate buffer (pH 7.0, 0.1M) (0.32 ml). To the suspension was added 7% aqueous acrylonitrile solution (0.08 ml), and the mixture was allowed to react at 30°C for one hour. As a result, it was found that acrylonitrile in the reaction mixture was thoroughly converted into acrylamide.

Example 14

The reaction in Example 13 was repeated using a similar method except that Erwinia nigrifluens MAFF 03-01435 was used instead of the JCM 1204 strain in Example 13. As a result, it was found that acrylonitrile in the reaction mixture was thoroughly converted into acrylamide.

**Claims**

1. A method for preparing amides from nitriles by an action of a microorganism, characterized in that said microorganism is a microorganism belonging to a genus of Klebsiella, Aeromonas, Citrobacter, Agrobacterium, Rhizobium, Xanthobacter, Erwinia, Enterobacter or Streptomyces and having an ability of converting nitriles to amides.

2. The method according to Claim 1 wherein said nitriles are added to a culture medium of said microorganism.

3. The method according to Claim 2 wherein said nitriles are added continuously or intermittently.

4. The method according to Claim 1 wherein said nitriles are acrylonitrile.

5. The method according to Claim 1 wherein said microorganism is Klebsiella sp. MCI 2609, Aeromonas sp. MCI 2614, Citrobacter freundii MCI 2615, Rhizobium sp. MCI 2610, Rhizobium sp. MCI 2643, Agrobacterium rhizogenes IAM 13570, Agrobacterium tumefaciens IAM 13129, Rhizobium loti IAM 13588, Rhizobium legminosarum IAM 12609, Rhizobium melioti IAM 12611, Xanthobacter flavus JCM 1204, Erwinia nigrifluens MAFF 03-01435, Enterobacter sp. MCI 2707, or Streptomyces sp. MCI 2691.

6. Klebsiella sp. MCI 2609 (FERM BP-3949).

7. Aeromonas sp. MCI 2614 (FERM BP-3950).

8. Citrobacter freundii MCI 2615 (FEB BP-3951).

9. Rhizobium sp. MCI 2610 (FERM BP-3952).

10. Rhizobium sp. MCI 2643 (FERM BP-3953).

11. Enterobacter sp. MCI 2707 (FERM BP-3955).

12. Streptomyces sp. MCI 2691 (FERM BP-3954).